# EUROPEAN PATENT APPLICATION

(11) **EP 1 953 658 A1**
(43) Date of publication of application: **06.08.2008**
(21) Application number: 07075017.9
(22) Date of filing: 09.01.2007
(51) Int. Cl.: G06F 19/00

(54) **Method for estimating a breeding value for an organism without a known phenotype**

(71) Applicant: ASG Veehouderij B.V., 8219 PH Lelystad (NL)
(72) Inventor: Calus, Mario Pieter Lea, 4524 KN Sluis (NL); Meuwissen, Theodorus Hendrikus Elisabeth, 1430 As (NO); Windig, Johannes Jacob, 8224 GA Lelystad (NL); Veerkamp, Roel Franciscus, 7364 AN Lieren (NL)
(74) Representative: van Loon, C.J.J.

(57) **Abstract**

Method for estimating a breeding value for a first organism without a known phenotype, comprising estimating a plurality of reference breeding values for a plurality of haplotypes of a plurality of second organisms, wherein a phenotype and a genotype of each of the second organisms of the plurality of second organisms are at least partially known, storing the plurality of estimated reference breeding values for the plurality of haplotypes, and estimating the breeding value for the first organism on the basis of at least a part of a genotype of the first organism and the stored plurality of estimated reference breeding values for the plurality of haplotypes.

## Description

The invention relates to a method for estimating a breeding value for a first organism without a known phenotype comprising estimating a plurality of reference breeding values for a plurality of haplotypes of a plurality of second organisms, wherein a phenotype and at least one haplotype of each of the second organisms of the plurality of second organisms are at least partially known, and estimating the breeding value for the first organism on the basis of at least a part of a genotype of the first organism and the plurality of estimated reference breeding values for the plurality of haplotypes.

The invention further relates to a computer program product including program code portions for performing, when run on a programmable apparatus, a method according to the invention, and to a computer readable medium comprising data representing a computer program product according to the invention.

Such method is for instance known from the paper of T.H.E. Meuwissen, B.J. Hayes and M.E. Goddard, 2001, "Prediction of Total Genetic Value Using Genome-Wide Dense Marker Maps", Genetics 157:1819-1829. The method described by Meuwissen et *al.,* however, has the disadvantage that it requires large amounts of computer (memory) resources and/or long calculation times to yield an estimated breeding value for a, e.g. juvenile, organism of which no phenotype is known (yet). Hence, it is not possible to quickly estimate a breeding value for such organism, whenever such is desired.

It is an object of the invention to at least diminish the above problem.

Thereto, according to a first aspect of the invention a method according to claim 1 is provided.

According to the first aspect of the invention, the plurality of estimated reference breeding values for the plurality of haplotypes of the plurality of second organisms is stored, e.g. in a non-volatile memory such as a computer memory and/or a database. This allows the plurality of reference breeding values to be estimated, e.g. calculated, when and/or where this is convenient, regardless of at that time and/or place a breeding value for a first organism of which a phenotype is not known (yet) needs to be estimated. The breeding value for a certain first organism of which a phenotype is not known (yet) can be estimated, e.g. calculated, separately from estimation of the stored plurality of estimated reference breeding values, when and/or where this is desired, on the basis of at least a part of a genotype of the first organism and on the basis of the stored plurality of estimated reference breeding values for the plurality of haplotypes.

Preferably, the plurality of reference breeding values for the plurality of haplotypes based on the plurality of second organisms is estimated and stored repeatedly at first moments in time, e.g. when the number of (partially phenotyped and genotyped) second organisms of the plurality of second organisms has increased, e.g. with such a number of second organisms that renewed calculation of the plurality of reference breeding value may yield more accurate reference breeding values.

Preferably the breeding value for mutually different first organisms of a plurality of first organisms without the known phenotype is estimated at second moments in time. Preferably, at least one second moment in time does not coincide with any first moment in time. Preferably, the first moments in time are in average further apart than second moments in time. Hence, the plurality of reference breeding values is estimated at a lower repetition rate than that the breeding value of first organisms is estimated. Thus, the procedure which requires the most computation time, and/or computer memory, i.e. estimating the plurality of reference breeding values, is not performed every time the breeding value of an individual first organism or a plurality of first organisms is estimated, thus reducing the total amount of computation time, and/or computer memory requirement and/or decreasing the time required to estimate the breeding value of first organisms.

According to a second aspect of the invention, estimating the plurality of reference breeding values for the plurality of haplotypes of the plurality of second organisms comprises deriving a relationship matrix comprising relationships between second organisms, based on the pedigree of the second organisms, determining for each of the second organisms in the matrix whether marker alleles of the at least one haplotype were inherited from its sire or its dam, deriving for each of the second organisms in the matrix at least one haplotype, each corresponding to at least one marker allele of that second organism, linking phenotypes of at least some of the second organisms to haplotypes of at least some of the second organisms, and estimating the reference breeding value for the at least one haplotype. Hence, the reference breeding values are estimated in a very efficient manner.

Herein haplotype may be defined as the allele of a single marker locus. Alternatively, haplotype may be defined as a combination of alleles of at least two neighbouring marker loci. The method according to the invention may comprise calculating, for at least one locus, an IBD-matrix comprising probabilities that haplotypes for that locus occurring in mutually different second organisms (or within a single second organism), are identical-by-descent, and calculating an inverted IBD-matrix from the IBD matrix by matrix inversion.

According to a third aspect of the invention, the method, e.g. a computer program arranged for performing the steps of the method, is arranged to allow a user of the method to choose whether the haplotype is defined as the allele of a single marker or as a combination of alleles of at least two neighbouring markers and/or whether or not an inverted IBD-matrix is calculated. This provides the user with control of the method, and allows to tailor the definition of the haplotype and the use of the inverted IBD-matrix to the specific needs, e.g. to the known haplotype of the first organism.

According to a fourth aspect of the invention, the method comprises storing the inverted IBD-matrix in a non-volatile memory, such as a computer memory. Preferably, the method comprises discarding zero-value elements, and/or diagonal elements, of the inverted IBD-matrix and storing non-zero, and/or off-diagonal, elements of the inverted IBD-matrix together with information representative of the positions of the non-zero, and/or off-diagonal, elements in the inverted IBD-matrix. Hence, it is possible to reduce the amount of data stored in the computer memory, while it is still possible to recreate the inverted IBD-matrix based on the stored non-zero, and/or off-diagonal, elements and the information representative of the positions of the non-zero, and/or off-diagonal, elements in the inverted IBD-matrix.

According to a fifth aspect of the invention, the method comprises storing an incidence matrix, linking phenotypes to haplotypes, in a non-volatile memory, such as a computer memory. Preferably, the method comprises discarding zero-value elements of the incidence matrix and storing non-zero elements of the incidence matrix together with information representative of the positions of the non-zero elements in the incidence matrix. Hence, it is possible to reduce the amount of data stored in the computer memory, while it is still possible to recreate the incidence matrix based on the stored non-zero elements and the information representative of the positions of the non-zero elements in the incidence matrix.

According to a sixth aspect of the invention, haplotypes having an identical-by-descent probability larger than a predetermined value are grouped as a single haplotype, i.e. are given an identical-by-descent probability of 100%. This reduces the amount of different haplotypes to be used in the estimation of the breeding value, as haplotypes which have a high probability to be identical-by-descent, viz. higher than the predetermined value, are in the estimation treated as being identical, thus being one single haplotype.

Preferably, the predetermined value is 65%, preferably 80%, more preferably 95%.

According to a seventh aspect of the invention, the reference breeding value for the one or more haplotypes is estimated using Gibbs sampling. Preferably, Gibbs sampling is performed using the sparse matrix technique, e.g. using the inverted IBD-matrix of which only non-zero and/or off-diagonal elements are stored together with the information representative of the positions of the non-zero and/or off-diagonal elements. This provides the advantage that the amount of computing time and/or memory requirement for performing the Gibbs sampling is reduced.

According to an eighth aspect of the invention, estimating the plurality of reference breeding values for the plurality of haplotypes comprises calculating coancestries between all haplotypes of first generation genotyped second organisms for each marker locus. Preferably, the coancestries are stored in a non-volatile memory, such as a computer memory. The coancestries between all haplotypes of first generation genotyped second organisms are needed for calculations at each marker locus. Thus, the coancestries may be calculated once and stored in the computer memory for use for each of the marker loci.

According to a ninth aspect of the invention, a method for selective breeding is provided, comprising selecting a sire and a dam, producing offspring of the sire and dam, estimating a breeding value of the offspring of the sire and dam using the method for estimating the breeding value for an organism without a known phenotype according to the invention, using the offspring as sire or dam for a next generation of offspring if the estimated breeding value offspring is equal to, or differs less than a predetermined amount from, a desired breeding value for the offspring. Preferably, the offspring is used as sire or dam before the phenotype associated with the estimated breeding value actually becomes manifest in the offspring.

The invention will now be elucidated by means of, non-limiting, examples and figures. Herein:
Fig. 1a illustrates an example of a flow chart which graphically demonstrates a first part of an example of the method according to the invention;
Fig. 1b illustrates an example of a flow chart which graphically demonstrates a second part of an example of the method according to the invention;
Fig. 2 illustrates a second example of a flow chart which graphically demonstrates a first part of an example of the method according to the invention;
Fig. 3a shows an example comprising two inverted IBD-matrices; and
Figs. 3b-3e show examples of arrays used in storing information representative of the inverted IBD-matrices of Fig. 3a.

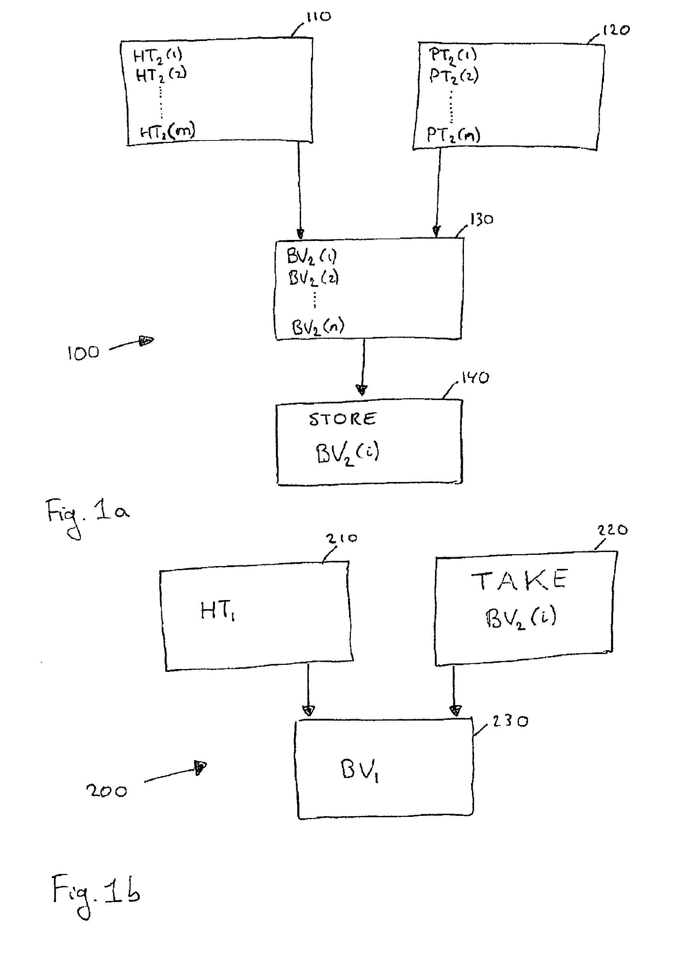

Fig. 1a illustrates an example of a flow chart which graphically demonstrates a first part 100 of an example of the method for estimating a breeding value BV₁ for a first organism O₁ without a known phenotype according to the invention. The first organism O₁ without the known phenotype may for example be a calf of which no milk yield is known yet, but of which an estimated milk yield needs to be known in order to decide whether or not to include the calf in a breeding program. Knowing the estimated breeding value before the actual phenotype can be known may reduce the time required for breeding programmes, since the first organism can be used in a breeding programme on the basis of the estimated breeding value before the phenotype actually becomes manifest.

The first part 100 relates to estimating a plurality of reference breeding values BV₂(i) (i=1,2,3,...) for a plurality of haplotypes HT₂(i) of a plurality of second organisms O₂(i). The plurality of second organisms O₂(i) may for instance comprise cows of which a (partial) haplotype is known and of which a (partial) phenotype is known, e.g. of which the milk yield is known. It will be appreciated that the first part 100 may be performed by a computer program running on a programmable computer.

In step 110 a genotype, in this example the haplotypes HT₂(i), of each second organism O₂(i) of the plurality of second organisms is determined. In this example each second organism O₂(i) has two haplotypes HT₂(i) per marker locus. Herein entering a haplotype determined in a manner known per se into a (e.g. non-volatile) memory, a computer, computer memory and/or database is also considered to be determining the haplotype as indicated in step 110.

In step 120 a phenotype PT₂(i) of each second organism O₂(i) of the plurality of second organisms is determined. Herein entering a phenotype determined in a manner known per se into a (e.g. non-volatile) memory, a computer, computer memory and/or database is also considered to be determining the phenotype as indicated in step 120. It will be appreciated that the phenotype PT₂(i) and the haplotypes HT₂(i) of each of the second organisms O₂(i) of the plurality of second organisms are at least partially known.

In step 130 the plurality of reference breeding values BV₂(i) is estimated, e.g. using a Gibbs sampler for Gibbs sampling. In each iteration the Gibbs sampler samples whether a marker locus is associated with a quantitative trait locus (QTL) or not, and estimates the breeding value of the haplotypes associated with the marker locus, depending on whether the marker locus is associated with a QTL or not. In step 130 a reference breeding value BV₂(i) is estimated corresponding to the haplotypes HT₂(i) of each second organism of the plurality of second organisms O₂(i), on the basis of the phenotype PT₂(i) associated with that second organisms O₂(i). Hence, in this example in step 130 phenotypes of at least some of the second organisms are linked to known haplotypes of at least some of the second organisms. It will be appreciated that it is also possible to estimate a reference breeding value BV₂(i) for each haplotype of the plurality of haplotypes HT₂(i) of the second organisms and/or for each possible combination of haplotypes of the second organisms.

The step 130 may comprise determining an incidence matrix, linking phenotypes to haplotypes. The incidence matrix may be stored, e.g. in a non-volatile memory, such as a computer memory. In order to reduce the amount of data to be stored, zero-value elements of the incidence matrix may be discarded before storage. Non-zero elements of the incidence matrix may be stored together with information representative of the positions of the non-zero elements in the incidence matrix.

The step 130 of estimating the plurality of reference breeding values BV₂(i) may comprise calculating coancestries between all haplotypes of first generation genotyped second organisms for each marker locus, as the expected identical-by-descent probability is larger for two haplotypes of organisms with common ancestors in the known pedigree [. It is possible that the calculated coancestries are stored, e.g. in a non-volatile memory such as a computer memory. Since these coancestries are required for calculations of identical-by-descent probabilities of haplotypesat each marker locus due to common ancestors in the known pedigree, retrieving stored coancestries from the non-volatile memory instead of calculating coancestries when required may reduce computing time.

For first generation genotyped second organisms the origin (sire or dam) of alleles at a marker locus cannot be determined. Hence, coancestries between pairs of haplotypes of first generation genotyped second organisms may be averaged, i.e. the probability that a certain allele is inherited from the sire or the dam is assumed to be equal.

In step 140 the plurality of estimated reference breeding values BV₂(i) for the plurality of haplotypes is stored, e.g. in a non-volatile memory such as a computer memory. It will be appreciated that the computer memory wherein the plurality of estimated reference breeding values BV₂(i) is stored may be a database. The plurality of estimated reference breeding values BV₂(i) may e.g. be stored for future use, future reference, use by others than those who gathered and/or entered the plurality of estimated reference breeding values BV₂(i) in the computer memory, and/or remote use, i.e. use on a location which is different from the location at which the memory is physically present.

Fig. 1b illustrates an example of a flow chart which graphically demonstrates a second part 200 of an example of the method according to the invention. The second part 200 relates to estimating the breeding value BV₁ for the first organism O₁. It will be appreciated that the second part 200 may be performed by a computer program running on a programmable computer.

In this example the first organism O₁ belongs to a same breeding population as the plurality of second organisms O₂, i.e. in this example at least one of the parents of the first organism, preferably both parents, is a second organism of the plurality of second organisms. Thus, the first organism has the same breeding and selection history as the second organisms, i.e. has the same genetic source. Hence, a haplotype of the first organism O₁ may be identical by descent to a haplotype of at least one of the second organisms of the plurality of second organisms.

In step 210 a genotype, in this example a pair of haplotypes, HT₁ of the first organism O₁ is determined. Herein entering a genotype determined in a manner known per se into a (e.g. non-volatile) memory, a computer, computer memory and/or database is also considered to be determining the genotype as indicated in step 210. The first organism O₁ may for instance be a juvenile organism of which no phenotype is known yet. In step 220 the plurality of estimated reference breeding values BV₂(i) is taken, e.g. retrieved, from the non-volatile computer memory. In step 230 the breeding value BV₁ for the first organism O₁ is estimated on the basis of at least a part of a genotype of the first organism, e.g. (one of) the haplotypes HT₁, and on the basis of the plurality of estimated reference breeding values BV₂(i) for the plurality of haplotypes HT₂(i). Herein the relation of the determined haplotype of the first organism is associated to all haplotypes of the second organisms on the same locus.

The exemplary method as depicted in Fig. 1a and 1b allows the plurality of reference breeding values BV₂(i) to be estimated, e.g. calculated, when and/or where this is convenient. Next, the breeding value BV₁ for the first organism O₁ of which a phenotype is not known (yet) can be estimated (in part 200), e.g. calculated, separately from the estimation of the plurality of reference breeding values BV₂(i) (in part 100), when and/or where this is desired, and, in this example, on the basis of at least a part of the haplotypes HT₁ of the first organism and the stored plurality of estimated reference breeding values BV₂(i) for the plurality of haplotypes HT₂(i) of the plurality of second organisms O₂(i).

In a special embodiment the first part 100, i.e. estimating the plurality of reference breeding values BV₂(i) for the plurality of haplotypes HT₂(i) of the plurality of second organisms O₂(i), is performed more than once. The first part 100 may for instance be repeated if a predetermined number of second organisms can be added to the plurality of second organisms O₂(i), which addition may increase the accuracy of the estimation of the plurality of reference breeding values BV₂(i) from the thus enlarged plurality of haplotypes of the enlarged plurality of second organisms. Thus the stored plurality of reference breeding values BV₂(i) may be updated. The first part 100 may for instance be repeatedly performed at first moments in time. These first moments may be spaced irregularly in time, e.g. when the first moments are determined by the number of second organisms to be added to the plurality of second organisms. The first moments may also be spaced substantially regularly in time. The first part 100 may e.g. be performed every six months.

The second part 200, i.e. estimating the breeding value BV₁ for the first organism O₁ without the known phenotype is performed at a second moment in time, or may be performed more than once, at a plurality of second moments in time. The second part 200 may be performed for a plurality of first organisms O₁(j) (j=1,2,3...) at a single second moment in time or at a plurality of second moments in time. In a special embodiment the second part 200 is performed whenever it is desired, e.g. at the moment there is a need to estimate the breeding value of the first organism.

Since the plurality of reference breeding values BV₂(i) is stored, e.g. in the non-volatile memory, there is no need to estimate the breeding value BV₁ for the first organism O₁ on a first moment in time. In fact, at least one second moment in time at which the breeding value BV₁ for a first organism is estimated may not coincide with any first moment in time. Thus the calculation time required for estimating the breeding value BV₁ for the first organism can be relatively short. In a special embodiment the first moments in time are in average further apart than the second moments in time. Hence, in this example the plurality of estimated reference breeding values BV₂(i) stored in the memory may be updated less frequently in time than that a breeding value BV₁ is estimated for a first organism O₁. Hence, the first part 100 of the method, which requires the larger part of the total computing time, may be performed less regularly than the second part 200, thus reducing the total computing time for the method.

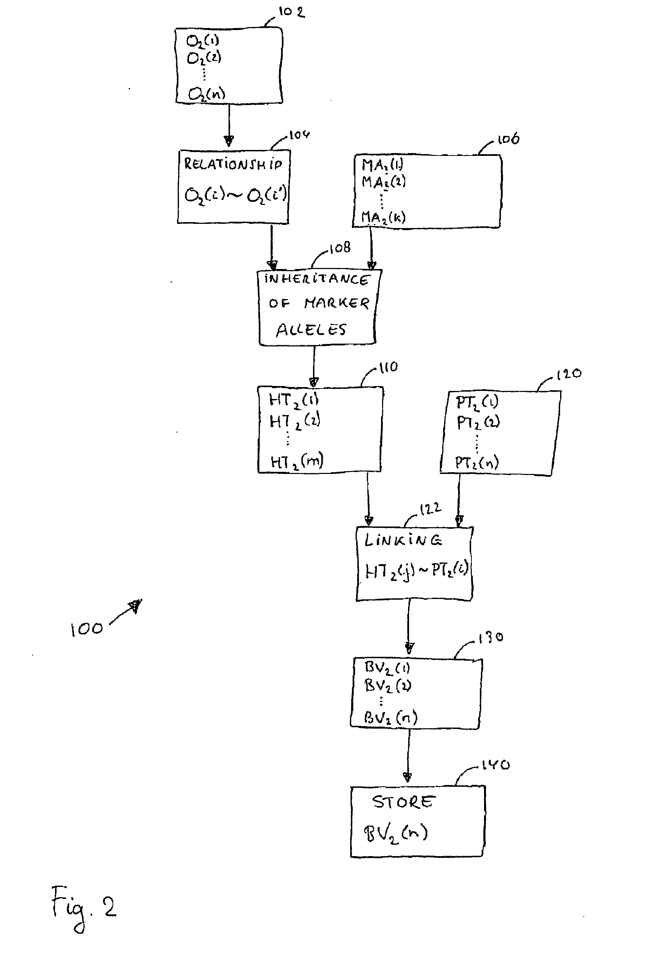

Fig. 2 illustrates a second example of a flow chart which graphically demonstrates the first part 100 of an example of the method according to the invention. In step 102 a plurality of second organisms O₂(i) is determined. Herein entering an identification for each second organism into a (e.g. non-volatile) memory, a computer, computer memory and/or database is also considered to be determining the plurality of second organisms as indicated in step 102. In step 104 the relationships based on pedigree between all second organisms of the plurality of second organisms is determined, i.e. for each combination of two second organisms of the plurality of organisms it is determined if, and if yes how, the two second organisms are related to each other, based on pedigree. The relationships may e.g. be stored in a relationship matrix, which may be stored in a non-volatile memory, such as a computer memory and/or a database, and may e.g. be updated on the first moments in time. Herein entering the relationships into a (e.g. non-volatile) memory, a computer, computer memory and/or database is also considered to be determining the relationships as indicated in step 104.

In step 106 for each second organism O₂(i) marker alleles MA₂(i) (alleles of a marker locus) are determined. Herein entering a marker allele determined in a manner known per se into a (e.g. non-volatile) memory, computer, computer memory and/or database is also considered to be determining the marker allele as indicated in step 106.

Next, in step 108 for each of the second organisms O₂(i) it is determined, on the basis of the relationships determined in step 104 and the marker alleles determined in step 106, whether marker alleles of the second organism O₂(i) were inherited from the sire or the dam of that second organism.

In step 110 for each second organism O₂(i) one or more haplotypes HT₂(i) are derived, each corresponding to one or more of the marker alleles MA₂(i). The haplotype HT₂(i) may be defined as as the allele of a single marker locus (marker allele). Alternatively, the haplotype may defined as a combination of alleles of at least two neighbouring marker loci. The haplotype may for example be defined as a combination of alleles of a plurality of marker loci. The first haplotype of the i^{th} second organism(e.g. i=1 for the first second organism) may be denoted as O₂(i):Locus₁Allele₁Locus₂Allele₁...LocusₘAllele₁, and a second haplotype of the i^{th} second organism may be denoted as O₂(i):Locus₁Allele₂Locus₂Allele₂..LocusₘAllele₂. For each second organism the first and second allele for a locus or for a plurality of loci may be determined using techniques known per se.

In step 120 the phenotype PT₂(i) of each second organism O₂(i) of the plurality of second organisms is determined as explained with respect to Fig. 1a. In step 122 for at least some of the second organisms, in this example for each second organism, the phenotype PT₂(i) as determined in step 120 is linked to the haplotype HT₂(i) as determined in step 110. Note that if polygenic effects are taken into account in step 122, the relationships determined in step 104 may be used.

Next, In step 130 the reference breeding value BV₂(i) is estimated for each haplotype HT₂(i) of the plurality of second organisms O₂(i), on the basis of the phenotype PT₂(i) associated with second organisms O₂(i) of the plurality of second organisms.

In a special embodiment, the method may further comprise calculating, for at least one locus an Identical-By-Descent-matrix (IBD-matrix) comprising identical-by-descent probabilities, i.e. probabilities that haplotypes HT₂(i) for that at least one locus occurring in mutually different second organisms (or within one second organism), are identical-by-descent. An inverted IBD-matrix may be generated from the IBD-matrix using matrix inversion. The inverted IBD-matrix may for instance be calculated in step 108. The identical-by-descent probability, denoted as P(IBD), may be calculated on the basis of haplotypes of marker alleles of the mutually different second organisms. Alternatively, or additionally, the identical-by-descent probability may be calculated on the basis of pedigree information of the mutually different second organisms.

The identical-by-descent probability based solely on marker alleles, assuming common ancestors with a certain probability due to finite population size and history, may be denoted as P(IBD | markers). The identical-by-descent probability based on markers and pedigree may be denoted as P(IBD | marker, pedigree). The identical-by-descent probability based on marker alleles and pedigree may be calculated as P(IBD)= P(IBD | marker, pedigree) + (1-P(IBD | marker, pedigree))*P(IBD | markers).

In order to reduce the number of haplotypes HT₂(i) used to estimate the breeding value of the first organism O₁, and hence reduce the computing time and/or memory requirements for estimating the breeding value BV₁, it is possible to group together haplotypes HT₂(i) having an identical-by-descent probability which is larger than a predetermined value, i.e. the haplotypes HT₂(i) having an identical-by-descent probability which is larger than a predetermined value are given an identical-by-descent probability of 100%. Preferably the predetermined value is 65%, more preferably 80%, most preferably 95%.

Preferably, the method comprises skipping calculation of the identical-by-descent probability based on pedigree information for haplotypes of two unrelated second organisms. Thus, for instance the computer program arranged to perform the method according to the invention may be arranged to determine whether or not two second organisms are related, e.g. from relationships determined in step 104. The computer program may then calculate the identical-by-descent probability if the two second organisms are related, and may refrain from calculating the identical-by-descent probability if the two second organisms are not related. This reduces computing time in case the two second organisms are not related.

In a special embodiment, the method comprises storing the inverted IBD-matrix in a non-volatile memory such as a computer memory and/or a database. Hence, for known second organisms, the inverted IBD-matrix and/or identical-by-descent probabilities need to be calculated only once.

The inverted IBD-matrix may comprise many elements, so that storing the inverted IBD-matrix in the non-volatile memory may require much storage space. According to a further aspect of the invention zero-value elements of the inverted IBD-matrix are discarded before storing the inverted IBD-matrix, while the non-zero elements of the inverted IBD-matrix are stored together with information representative of the positions of the non-zero elements in the inverted IBD-matrix. Additionally the diagonal elements of the inverted IBD-matrix may be discarded before storing the inverted IBD-matrix. This allows to reduce the amount of memory required to store the inverted IBD-matrix.

It will be appreciated that the first part 100 shown in Fig. 2 can be used in combination with the second part 200 shown in Fig. 1b as explained with respect to Figs. 1a and 1b.

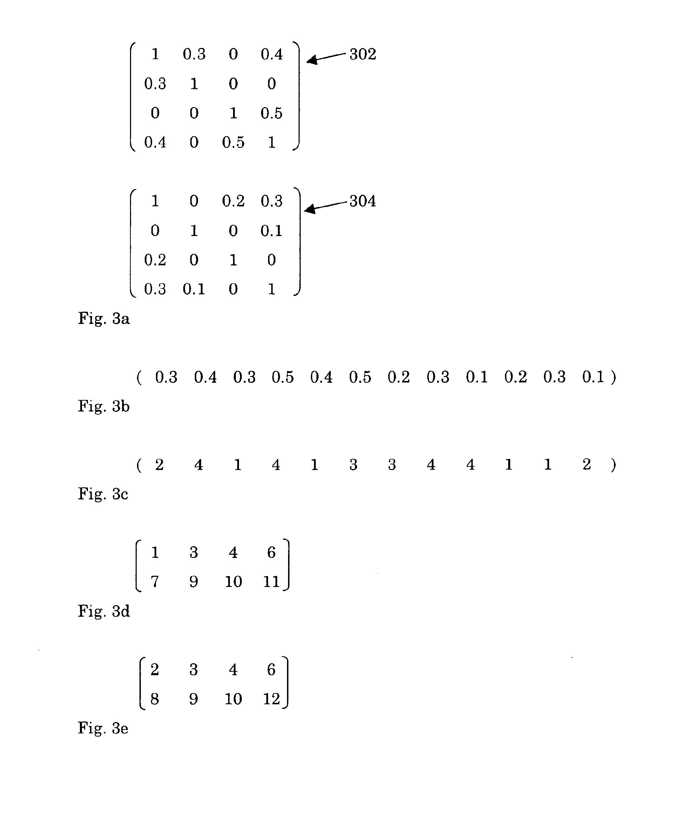

Fig. 3a shows an example comprising two inverted IBD-matrices 302 and 304. In this example the non-zero off-diagonal elements of the inverted IBD-matrices are stored in a first linear array A₁(p) (p=1,2,3,...), shown in Fig. 3b, starting with the non-zero off-diagonal values of the first row of the inverted IBD-matrix 302, followed by the non-zero off-diagonal values of the second and each subsequent row, for both inverted IBD-matrices 302 and 304. In a second linear array A₂(p), shown in Fig. 3c, having the same number of elements as the first linear array A₁(p), each element comprises the number of the column of the inverted IBD-matrix in which the corresponding non-zero off-diagonal element in the first linear array A₁(p) was originally present. Fig. 3d shows a two-dimensional third array A₃ containing the first positions in the linear arrays A₁(p) and A₂(p) of the rows of each inverted IBD-matrix. Each row in the third array A₃ represents and individual inverted IBD-matrix. Fig. 3e shows a two-dimensional fourth array A₄ containing the last positions in the linear arrays A₁(p) and A₂(_{P}) of the rows of each inverted IBD-matrix. Each row in the fourth array A₄ represents and individual inverted IBD-matrix. From the four arrays A₁(p), A₂(p), A₃ and A₄ the original inverted IBD-matrices 302,304 can be reconstructed. Further, from the four arrays A₁(p), A₂(p), A₃ and A₄ a Gibbs-sampler, which requires all non-zero off-diagonal elements from a row in an inverted IBD-matrix at the same time, can easily retrieve all non-zero off-diagonal elements from a row in the inverted IBD-matrix.

With respect to Figs. 2 and 3a-3e it has been explained that identical-by-descent probabilities are calculated for second organisms O₂(i). It will be appreciated that it is also possible to calculate for at least one of the haplotypes HT₁ of the first organism O₁ the identical-by-descent probability that that haplotype is identical to at least one haplotype HT₂(i) of at least one of the second organisms O₂(i).

The method may be arranged to allow a user of the method to choose whether the haplotype is defined as the allele of a single marker locus or as a combination of alleles of at least two neighbouring marker loci and/or whether or not an inverted IBD-matrix is calculated. Hence, the user can use the desired definition of haplotype. The method defining haplotypes as the allele of a single marker locus may be used in a backcross population, where only two genotypes are segregating, while the method defining haplotypes based on multiple marker alleles including inverted IBD-matrices, might be used in outbred populations. The method may for instance be incorporated in a computer program to be run on a programmable computer, wherein the program is arranged to allow the user of the program to choose whether the haplotype is defined as the allele of a single marker locus or as a combination of alleles of at least two neighbouring marker loci and/or whether or not an inverted IBD-matrix is calculated.

The method of estimating the breeding value for the first organism without a known phenotype according to the invention, may be used in a method for selective breeding. According to an aspect of the invention, the method for selective breeding, here also referred to as breeding program, comprises selecting a sire and a dam, e.g. from the plurality of second organisms, respectively having breeding values BV_{S} and BV_{D}. The breeding values BV_{S} and BV_{D} are chosen, in a manner known per se, such that offspring of the sire and dam may have a desired breeding value BV_{des}. Next, offspring is produced using the sire and dam. The first organism here is offspring of the sire and dam. The breeding value BV₁ of the first organism may be estimated as decribed hereinabove, e.g. before the phenotype, associated with the desired breeding value BV_{des}, becomes manifest in the first organism. The breeding program may proceed with the first organism as sire or dam for a next generation of offspring if the estimated breeding value BV₁ of the first organism is equal to, or differs less than a predetermined amount from, the desired breeding value BV_{des} for the first organism. Thus, knowing the estimated breeding value before the actual phenotype can be known may reduce the time required for the breeding program, since the first organism can be used in the breeding program on the basis of the estimated breeding value before the phenotype associated with the estimated breeding value actually becomes manifest in the first organism.

The method according to the invention may be embodied in a computer program product including program code portions for performing, when run on a programmable apparatus, the method. Data representing the computer program product may be stored on a computer readable product, comprising, but not limited to, storage media such as magnetic storage media (ROMs, RAMs, floppy discs, magnetic tapes, etc.), optically readable storage media (CD-ROMs, DVDs, etc.), and carrier waves (transmission via the internet). Further, the computer program product may be implemented in a distributed fashion, e.g. comprising a first portion, e.g. for performing the first part 100, and a second portion, e.g. for performing the second part 200, wherein the first and second portions may be arranged to be run on mutually different programmable apparatus and/or at mutually different (remote) locations.

In the foregoing specification, the invention has been described with reference to specific examples of embodiments of the invention. It will, however, be evident that various modifications and changes may be made therein without departing from the broader spirit and scope of the invention as set forth in the appended claims.

For example, the Gibbs sampling referred to with respect to step 130 may be performed using the sparse matrix technique for a further reduction of computing time and/or memory requirement.

In the example of Figs 3a-3e a plurality of inverted IBD-matrices is stored in the four arrays A₁-A₄. It will be appreciated that it is also possible to store a single inverted IBD-matrix in the four arrays.

It will be appreciated that estimating the plurality of reference breeding values may also take into account polygenic effects.

It will be appreciated that estimating the breeding value for the first organism may also be based on pedigree of the first organism and/or take into account polygenic effects.

However, other modifications, variations and alternatives are also possible. The specifications, drawings and examples are, accordingly, to be regarded in an illustrative rather than in a restrictive sense.

In the claims, any reference signs placed between parentheses shall not be construed as limiting the claim. The word 'comprising' does not exclude the presence of other features or steps then those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to advantage.

## Claims

1. Method for estimating a breeding value for a first organism without a known phenotype, comprising:
estimating a plurality of reference breeding values for a plurality of haplotypes of a plurality of second organisms, wherein a phenotype and at least one haplotype of each of the second organisms of the plurality of second organisms are at least partially known,
storing the plurality of estimated reference breeding values for the plurality of haplotypes, and
estimating the breeding value for the first organism on the basis of at least a part of a genotype of the first organism and the stored plurality of estimated reference breeding values for the plurality of haplotypes.

2. Method according to claim 1, wherein the breeding value for the first organism is estimated on the basis of at least one haplotype of the first organism and the stored plurality of estimated reference breeding values.

3. Method according to claim 1 or 2, wherein the plurality of estimated reference breeding values for the plurality of haplotypes is stored in a non-volatile memory such as a computer memory and/or a database.

4. Method according to claim 3, wherein the non-volatile memory is remote from a location where the breeding value for the first organism is estimated.

5. Method according to any one of the preceding claims, wherein estimating the plurality of reference breeding values for the plurality of haplotypes based on the plurality of second organisms and storing the plurality of estimated reference breeding values is repeatedly performed at first moments in time.

6. Method according to any one of the preceding claims, wherein estimating the breeding value for mutually different first organisms of a plurality of first organisms without the known phenotype is performed at second moments in time.

7. Method according to claim 5 and 6, wherein at least one second moment in time does not coincide with any first moment in time.

8. Method according to claim 5 and 6, wherein the first moments in time are in average further apart than the second moments in time.

9. Method according to any one of the preceding claims, wherein estimating the plurality of reference breeding values for the plurality of haplotypes of the plurality of second organisms comprises linking phenotypes of at least some of the second organisms to known haplotypes of at least some of the second organisms.

10. Method according to any one of claims 1-8, wherein estimating the plurality of reference breeding values for the plurality of haplotypes of the plurality of second organisms comprises:
deriving a relationship matrix comprising relationships between second organisms, based on the pedigree of the second organisms;
determining for each of the second organisms in the matrix whether marker alleles of that second organism were inherited from its sire or its dam;
deriving for each of the second organisms in the matrix at least one haplotypes, each corresponding to at least one marker allele of that second organism;
linking phenotypes of at least some of the second organisms to haplotypes of at least some of the second organisms; and
estimating the reference breeding value for the at least one haplotype.

11. Method according to claim 9 or 10, comprising storing an incidence matrix linking phenotypes to haplotypes in a non-volatile memory, such as a computer memory.

12. Method according to any one of claims 1-11, wherein haplotype is defined as the allele of a single marker locus.

13. Method according to any one of claims 1-11, wherein haplotype is defined as a combination of alleles of at least two neighbouring marker loci.

14. Method according to any one of the preceding claims, further comprising calculating, for at least one marker locus an inverted IBD-matrix comprising probabilities that haplotypes for that locus are identical-by-descent.

15. Method according to claim 14, comprising calculating the identical-by-descent probability on the basis of marker haplotypes of the mutually different second organisms.

16. Method according to claim 14 or 15, comprising calculating the identical-by-descent probability on the basis of pedigree information of the mutually different second organisms.

17. Method according to claim 14 or 15, comprising skipping calculation of the identical-by-descent probability based on pedigree information for haplotypes of two unrelated organisms.

18. Method according to any one of claims 14-17, comprising storing the inverted IBD-matrix in a non-volatile memory.

19. Method according to claim 18, comprising discarding zero-value elements of the inverted IBD-matrix and storing non-zero elements of the inverted IBD-matrix together with information representative of the positions of the non-zero elements in the inverted IBD-matrix.

20. Method according to claims 12, 13 and 14, wherein the method is arranged to allow a user of the method to choose whether the haplotype is defined as the allele of a single marker locus or as a combination of alleles of at least two neighbouring marker loci and/or whether or not an inverted IBD-matrix is calculated.

21. Method according to claim 14, wherein haplotypes having an identical-by-descent probability larger than a predetermined value are grouped as a single haplotype, i.e. are given an identical-by-descent probability of 100%.

22. Method according to claim 21, wherein the predetermined value is 65%, preferably 80%, more preferably 95%.

23. Method according to at least claim 10, wherein the reference breeding value for the one or more haplotypes is estimated using Gibbs sampling.

24. Method according to claim 18, wherein Gibbs sampling is performed using the sparse matrix technique.

25. Method according to at least claim 2, comprising calculating for at least one haplotype of the first organism the identical-by-descent probability that the haplotype is identical to at least one haplotype of a second organism by descent.

26. Method according to any one of the preceding claims, wherein estimating the plurality of reference breeding values for the plurality of haplotypes comprises calculating coancestries between all haplotypes of first generation genotyped second organisms for each marker locus.

27. Method according to claim 26, comprising storing the coancestries in a non-volatile memory.

28. Method according to claim 26 or 27, comprising averaging coancestries between pairs of haplotypes of mutually different first generation genotyped second organisms.

29. Method for selective breeding, comprising
selecting a sire and a dam,
producing offspring of the sire and dam,
estimating a breeding value of the offspring of the sire and dam using a method according to any one of claims 1-28,
using the offspring as sire or dam for a next generation of offspring if the estimated breeding value of the offspring is equal to, or differs less than a predetermined amount from, a desired breeding value for the offspring.

30. Method according to claim 29, wherein the offspring is used as sire or dam before the phenotype associated with the estimated breeding value becomes manifest.

31. Computer program product including program code portions for performing, when run on a programmable apparatus, a method according to any one of claims 1-28.

32. Computer readable medium comprising data representing a computer program product according to claim 31.
